# EUROPEAN PATENT APPLICATION

(11) **EP 3 422 224 A1**
(43) Date of publication of application: **02.01.2019**
(21) Application number: 17179119.7
(22) Date of filing: 30.06.2017
(51) Int. Cl.: G06F 19/00

(54) **AN APPARATUS AND ASSOCIATED METHODS FOR DETERMINING USER ACTIVITY PROFILES**

(71) Applicant: Nokia Technologies Oy, 02610 Espoo (FI)
(72) Inventor: Baykaner, Khan, Upper Cambourne, Cambridgeshire CB23 6BL (GB); Dubba, Krishna, Bishops Stortford CM23 3BE (GB)
(74) Representative: Potter Clarkson LLP

(57) **Abstract**

An apparatus configured to, based on: a received daily target activity level for a user; and a received user daily activity profile for the user, the user daily activity profile comprising activity features each comprising specific intensity and specific duration at intervals throughout at least a portion of a day to cumulatively provide a particular activity level for the day, the user daily activity profile based on one or more of: at least one previous user daily activity profile of the user; and at least one previous user daily activity profile of one or more comparable users; generate an adjusted user daily activity profile comprising adjusted activity features each comprising one or more of adjusted specific intensity and adjusted specific duration at intervals throughout at least a portion of a day to cumulatively provide the daily target activity level for the user; and output the adjusted user daily activity profile for use by the user.

## Description

### Technical Field

The present disclosure relates to apparatus and methods associated with user activity profiles and generating adjusted user activity profiles to help a user achieve an activity goal.

Some examples may relate to portable electronic devices, in particular, so-called hand-portable electronic devices which may be hand-held in use (although they may be placed in a cradle in use). Such hand-portable electronic devices include so-called Personal Digital Assistants (PDAs) and tablet PCs. Certain portable electronic devices may be wearable, such as on the wrist. The portable electronic devices/apparatus according to one or more disclosed example aspects/embodiments may provide one or more audio/text/video communication functions (e.g. telecommunication, videocommunication, and/or text transmission, Short Message Service (SMS)/ Multimedia Message Service (MMS)/emailing functions, interactive/non-interactive viewing functions (e.g. web-browsing, navigation, TV/program viewing functions), music recording/playing functions (e.g. MP3 or other format and/or (FM/AM) radio broadcast recording/playing), downloading/sending of data functions, image capture function (e.g. using a (e.g. in-built) digital camera), and gaming functions.

### Background

Recent developments in technology include devices to help a user monitor their activities. A user may wish to improve their activity or lifestyle and use such technology as an aid.

The listing or discussion of a prior-published document or any background in this specification should not necessarily be taken as an acknowledgement that the document or background is part of the state of the art or is common general knowledge.

### Summary

According to a first aspect, there is provided an apparatus comprising: at least one processor; and at least one memory including computer program code, the at least one memory and the computer program code configured to, with the at least one processor, cause the apparatus to: based on: a received daily target activity level for a user; and a received user daily activity profile for the user, the user daily activity profile comprising activity features each comprising specific intensity and specific duration at intervals throughout at least a portion of a day to cumulatively provide a particular activity level for the day, the user daily activity profile based on one or more of: at least one previous user daily activity profile of the user; and at least one previous user daily activity profile of one or more comparable users; generate an adjusted user daily activity profile comprising adjusted activity features each comprising one or more of adjusted specific intensity and adjusted specific duration at intervals throughout at least a portion of a day to cumulatively provide the daily target activity level for the user; and output the adjusted user daily activity profile for use by the user.

A comparable user may be a user having one or more demographic factors in common with the user. Demographic factors may include age, age range, gender, weight, activity level, daily routine, activity type, profession, income, geographical location, ethnicity and disability. Data relating to comparable users, including demographic details and recorded comparable user activity profiles, and data relating to the user such as user demographic details and previous user daily activity profiles, may be stored on a remote server, or cloud, for access e.g. by the apparatus (or be stored on the apparatus).

A "portion of a day" may be: 6 hours, 8 hours, 12 hours, the period the user is awake, the period between sunrise and sunset, or 24 hours, for example.

The activity features have a specific intensity and a specific duration in the sense of the intensity and duration having specific values for a given activity. It follows that the corresponding adjusted activity features have an adjusted intensity and/or adjusted duration, adjusted compared with the specific values of intensity and duration of the activity feature. For example, an activity feature may have a specific intensity I and specific duration t, and the adjusted version of this activity feature has an adjusted specific intensity of 1.2 times I, and an adjusted specific duration of 1.1 times t.

The particular activity level for the day, obtainable from the user daily activity profile, is particular in the sense that the activity level can be defined as a particular quantity for comparison with the target activity level for the user. For example, the particular activity level may be that 10,000 steps were recorded, or that three hours of study were performed (the target activity levels may therefore be, for example, recording 11,000 steps in a day, or performing three and a half hours of study).

The apparatus may be configured to generate the adjusted user daily activity profile using a machine-learning model.

The received user daily activity profile for the user, based on one or more of at least one previous user daily activity profile of the user, and at least one previous user daily activity profile of one or more comparable users, may be generated in a training phase for a machine learning model. That is, a machine learning model may be trained using at least one previous user daily activity profile (i.e. logged activity by the user), and/or at least one previous user daily activity profile of one or more comparable users (i.e. logged or modelled activity from "similar" users). The previous user daily activity profile(s) may be called training data.

Following training using such data, the model may be provided with a daily target activity level (a control value) for the user, and, using the training data, generate an adjusted user daily activity profile for the user. In some examples, the model, once trained, may be used to generate an adjusted user daily activity profile without receiving an input of activity data (e.g. a user daily input profile from the user or a comparable user). That is, the apparatus may not be provided with activity data (a user daily activity profile) as an input following training, and can generate an adjusted user daily activity profile as an output. In some examples, the model, once trained, may be used to generate an adjusted user daily activity profile following input of a user daily input profile (e.g. the previous day's activity logged for the user).

If a daily target activity level is used as input, then the model may generate an adjusted user daily activity profile according to the daily target activity level (e.g. providing a control value as input which is a target activity level causes the model to generate an adjusted user daily activity profile providing that target activity level). If no daily target activity level is used as input, then the model may generate an adjusted user daily activity profile according to the training data used to train the model. Providing no daily target activity level as input is equivalent to providing a daily target activity level as input which is equal to the particular activity level for the day of the received user daily activity profile(s) used as training data.

The apparatus may be configured to use a machine-learning model to:
identify one or more of the activity features in the user daily activity profile as candidates for adjustment based on one or more of at least one previous user daily activity profile of the user; and at least one previous user daily activity profile of one or more comparable users; and
adjust the one or more identified candidate activity features, in accordance with the daily target activity level for the user, to generate the adjusted user daily activity profile.

The machine learning model may determine that, for example, a user is regularly achieving their goals for a walking type activity, and then adjust the activity feature to include jogging and walking to make the activity a harder work out. As another example, the machine learning model may determine that a user is regularly falling short of achieving their desired calorie-burn for a running based activity feature, so it may slowly introduce an additional swimming activity feature to help the user burn the target calories which cannot be achieved by running alone.

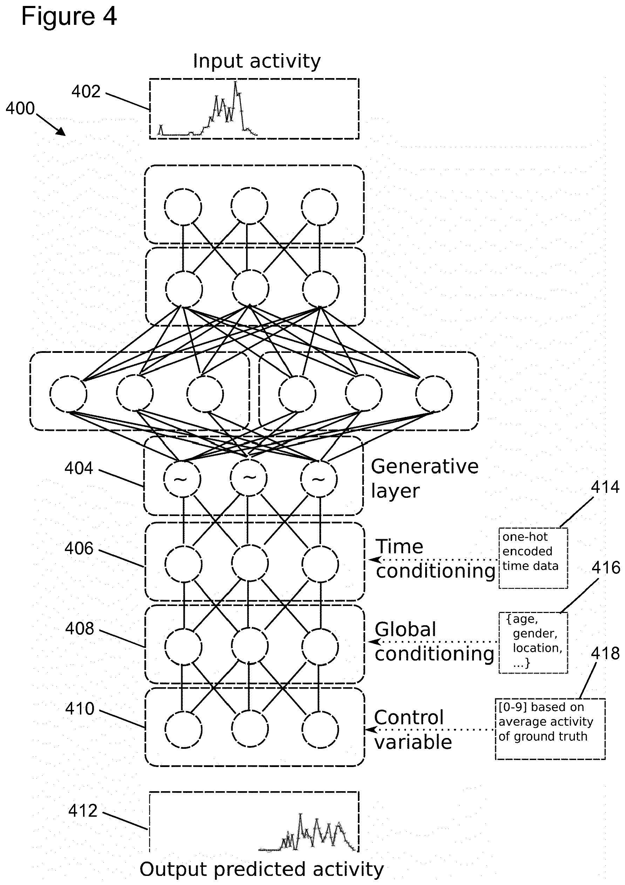

In some examples, to identify activity features as candidates for adjustment, the model may not explicitly classify the activity features. For example, the model discussed with reference to Figure 4 does not explicitly classify activity features (e.g. as a "swimming" peak, a "jogging" peak, etc.), but it does identify the correlations between the time conditioning information and the raw activity data. For example, the model may learn that at 2pm the user generally does 1 hour of intense exercise, and this identified activity feature, of an hour's intensive exercise at 2pm, can be incorporated into the prediction (the adjusted user daily activity profile) for the following day. So, identifying the one or more activity features may be based on all the conditioning variables fed in to the model (e.g. the demographics, the timing, activity intensity, and the control variable if used) in whatever organisation the model learns to best utilise these features (which may include totally ignoring irrelevant features).

In some examples, to identify activity features as candidates for adjustment, the model may explicitly classify the activity features. For example, the model may take annotated activities (and corresponding time of day that they happened) as a conditioning input (e.g. along with the global conditioning or in an additional layer). Then the model could learn to correlate the identification of 'swimming' with a particular level of activity.

Another example would be to use an activity classifier (e.g. another machine learning model) to generate activity type predictions. Such a model could take, for example, accelerometry, timing, wifi/radar, heart-rate and/or other data as input, and output the time and classes of activities (e.g. swimming from 2-3pm). These output classifications could then be fed into the activity generator model just as if they were annotations from a user.

To adjust the one or more identified candidate activity features, the activity model may generate a plausible looking output for a future activity profile (an adjusted user daily activity profile) given the input and conditioning variables (all of which may be present or absent). The model may be trained with a loss function that encourages the overall level of activity to be higher when the control variable is higher (hence the higher control variable causes an over-prediction of the current activity level for the day for the user), but may also be trained with a second loss function that aims to minimise the prediction error of the output activity profile. The model may learn that, for example, if a user is determined to increase their daily activity, they do it by going to the gym for two hours from 4-6pm, rather than, for example, by undertaking a small increase in activity at all times. This means that when the control variable is increased, the model will tend to try to generate data that looks like a two-hour trip to the gym. The model increases the intensity, and/or the duration of activity based on which activity profile is plausible compared with real data that the model was trained with, to produce an adjusted user daily activity profile with an e.g. higher overall activity level meeting the daily target activity level for the user. Plausibility of an adjusted activity feature may be determined by ensuring the adjusted activity feature lies within a predetermined confidence threshold (e.g. from a corresponding activity feature in the user daily activity profile) while the overall particular activity level for the day meets the daily target activity level (within a predetermined tolerance of e.g. ±5% of the daily target activity level).

The apparatus may be configured to generate the adjusted user daily activity profile by adjusting one or more of the specific intensity and specific duration of the activity features of the user daily activity profile evenly over the at least a portion of a day, in accordance with the daily target activity level for the user, to provide an adjusted user daily activity profile.

"Evenly" may be defined as each activity feature being adjusted by the same proportion (e.g. a five-point increase in heart rate) or relative proportion (e.g. 10% increase in heart rate) within a defined tolerance window (e.g. ± 10%.)

The apparatus may be configured to generate the adjusted user daily activity profile by adjusting activity features in the user daily activity profile to minimise the perceived change in daily activity by the user whilst still achieving the daily target activity level for the user.

Adjustments may be made by analysing one or more previous days of activity, from the user and/or from one or more other comparable users, and introducing the types of positive changes that are plausible given the activity patterns of the user and/or comparable user(s). For example, if a day on which the user does much more activity has a certain peak, that peak may be reproduced as an adjustment in an adjusted user daily activity profile if the control variable is increased (since the model may try to balance minimising reproduction error in the output activity profile with getting the overall activity level matched against the control variable).

The daily target activity level for the user may indicate an increase or decrease in activity level compared to at least one of: a particular activity level of at least one previous user daily activity profile of the user, and a particular activity level of at least one previous user daily activity profile of one or more comparable users.

An adjusted activity feature of the adjusted user daily activity profile may comprise one or more of:
the same specific intensity but an adjusted specific duration compared to a corresponding activity feature in the user daily activity profile;
the same specific duration but an adjusted specific intensity compared to a corresponding activity feature in the user daily activity profile; and
the same specific maximum intensity and same specific duration, but an adjusted gradient between minimum and maximum intensity levels of the adjusted activity feature compared to a corresponding activity feature in the user daily activity profile.

An adjusted activity feature may correspond to an activity feature by one or more of:
each occurring at the same time of day;
each relating to the same activity type, and
the adjusted activity feature providing the same percentage contribution to the daily target activity level for the user as the activity feature provides to the particular activity level of the user daily activity profile.

An adjusted activity feature may correspond to an activity feature by taking place at the same time of day (e.g. at 10am the user performs an activity for half an hour). An adjusted activity feature may correspond to an activity feature by being related to the same activity type (e.g. the user goes to the gym for an hour, but it doesn't matter when in the day the user does this). An adjusted activity feature may contribute the same level of activity to the daily target activity level as the corresponding activity feature contributes to the activity level of the user daily activity profile (e.g. 20% of target calories for the day burned, or 10% of the daily target having an activity level of "medium" if activities through the day are categorised as "low", "medium" or "high" activity level).

References to the "same" factor (e.g. activity intensity, activity duration, activity level percentage contribution for the day) may be defined as the same within a defined tolerance window of e.g. 2%, 5%, or 10%, for example.

Activity type may be categorised by: activity name (e.g. walking, gardening, swimming, tennis), activity intensity level (e.g. walking is level 1, jogging is level 2, swimming is level 3, circuit training is level 4), or by another grouping (e.g. cardio (running, circuit training), stretching (e.g. yoga, Pilates, Tai Chi), muscle-building (e.g. weightlifting), casual (e.g. outdoor walk, playing with grandchildren) or relaxing (sleeping, reading)).

The difference in the one or more of adjusted specific duration, adjusted specific intensity and adjusted gradient between minimum and maximum intensity levels of the adjusted activity feature compared with the corresponding one or more of specific duration, specific intensity and gradient between minimum and maximum intensity levels of the corresponding activity feature may be one or more of: less than 2%, 2%, 3%, 4%, 5%, 6%, 8%, 10%, and more than 10%.

The adjusted user daily activity profile may comprise an adjusted activity feature at a particular time of day corresponding to a time of no activity in the user daily activity profile. "No activity" may be defined as substantially zero, or negligible activity. For example, it may relate to an activity contributing to no more than a small (e.g. 5%) predefined percentage of the daily target activity level. Such times of no activity may be when the user is sleeping, reading, sitting still, or watching TV, for example. In other examples, sleeping may be considered to be the activity of interest, and a user may wish to aim for a target number of hours of sleep per day.

The apparatus may be configured to generate a series of adjusted user daily activity profiles which progressively provide an intermediate daily target activity level for the user over a predetermined plurality of days, the intermediate daily target activity level for the user varying progressively over the predetermined plurality of days until it equals the daily target activity level for the user.

Dependent on the user daily activity profile for the user for a particular day, the apparatus may be configured to adjust the daily target activity level for the user for a subsequent day to account for user activity recorded on the particular day. If the particular activity level for the particular day is higher than daily target activity level, the apparatus may be configured to increase the daily target activity level for the subsequent day. If the particular activity level for the particular day is lower than daily target activity level, the apparatus may be configured to decrease the daily target activity level for the subsequent day.

The apparatus may be configured to set the daily target activity level for the user according to a heuristic derived from activity data for one or more comparable users. The heuristic (or rule) may be derived from other users in experiments where a particular value (range) is demonstrated to encourage positive behaviour change in the user. For example, if a user does no activity between 9am and 11 am, but research shows this is a good time window to perform effective exercise, then adjusted activity features may be included according to the time criterion of 9am-11am. As another example, research may show that a particularly effective choice of activity for women over 40 to achieve their activity goal is yoga, so a "yoga" activity feature may be included for a 50-year-old woman in her user daily activity profile.

The user daily activity profile may be based on at least one previous user daily activity profile of one or more comparable users and not based on at least one previous user daily activity profile of the user. Such an apparatus may be configured to use an objective function to identify and reduce an error between the at least one previous user daily activity profile of one or more comparable users and the generated adjusted user daily activity profile. For example, the user may not have recorded any activity profiles yet with which to train the model.

The objective function may comprise one or more of:
a reproduction loss term configured to reduce an error between the generated adjusted user daily activity profile and a user daily activity profile received following output of the generated adjusted user daily activity profile;
a generative loss term configured to reduce an error in distributions between the at least one previous user daily activity profile of one or more comparable users and the generated adjusted user daily activity profile; and
a novel loss term configured to reduce an error between the particular activity level for the daily target activity level for the user obtained from the adjusted user daily activity profile, and the received daily target activity level.

The generative loss term in the objective function permits the application of a generative model without requiring an activity profile (of the user or a comparable user) to be provided as an input to the model when running the model to generate an adjusted user daily activity profile.

The apparatus may be configured to output the adjusted user daily activity profile for use by the user by one or more of:
providing for display of a graph indicating a current user daily activity profile and the target user daily activity profile;
providing a visual message prompting the user to one or more of perform or subsequently keep performing an upcoming activity in the adjusted user daily activity profile,
providing an audio message prompting the user to one or more of perform or subsequently keep performing an upcoming activity in the adjusted user daily activity profile, and
providing haptic feedback (e.g. vibration) prompting the user to one or more of perform or subsequently keep performing an upcoming activity in the adjusted user daily activity profile.

The apparatus may be configured to determine an activity type for an adjusted activity feature. Outputting the adjusted user daily activity profile for use by the user may comprise outputting the determined activity type to the user.

The user daily activity profile and the adjusted user daily activity profile may provide an indication of the variation of one or more of the following parameters with time:
calories used,
heart rate,
steps taken,
distance travelled,
body temperature,
body hydration and perspiration,
breathing rate,
blood oxygenation,
blood-sugar level,
completion status of a task, and
time asleep.

Body hydration and perspiration may be measured, for example, as electro-dermal activity / galvanic skin response.

Time spent studying may be measured as a "completion status of a task". For example, if a user wishes to study one chapter from a textbook each day, then the portion through the chapter which is studied at intervals through the day is indicated by a completion status of a task (e.g. for a 30-page chapter, after studying ten pages, the completion status of the study task is 33%).

The apparatus may be one or more of: an electronic device, a portable electronic device, a wearable device, a wristband device, a fitness tracker device, a smartwatch, smart eyewear, a portable telecommunications device, a mobile phone, a smartphone, a personal digital assistant, a tablet, a desktop computer, a laptop computer, a server, and a module for one or more of the same.

The apparatus may be configured to measure the one or more parameters indicated in the adjusted user daily activity profile (e.g. the apparatus may comprise a heart rate monitor, a pedometer or a thermal sensor).

According to a further aspect, there is provided a computer-implemented method comprising: based on a received daily target activity level for a user; and a received user daily activity profile for the user, the user daily activity profile comprising activity features each comprising specific intensity and specific duration at intervals throughout at least a portion of a day to cumulatively provide a particular activity level for the day, the user daily activity profile based on one or more of; at least one previous user daily activity profile of the user; and at least one previous user daily activity profile of one or more comparable users; generating an adjusted user daily activity profile comprising adjusted activity features each comprising one or more of adjusted specific intensity and adjusted specific duration at intervals throughout at least a portion of a day to cumulatively provide the daily target activity level for the user; and outputting the adjusted user daily activity profile for use by the user.

The steps of any method disclosed herein do not have to be performed in the exact order disclosed, unless explicitly stated or understood by the skilled person.

Corresponding computer programs for implementing one or more steps of the methods disclosed herein are also within the present disclosure and are encompassed by one or more of the described examples.

For example, in a further aspect there is provided a computer readable medium comprising computer program code stored thereon, the computer readable medium and computer program code being configured to, when run on at least one processor, perform the method comprising: based on a received daily target activity level for a user; and a received user daily activity profile for the user, the user daily activity profile comprising activity features each comprising specific intensity and specific duration at intervals throughout at least a portion of a day to cumulatively provide a particular activity level for the day, the user daily activity profile based on one or more of; at least one previous user daily activity profile of the user; and at least one previous user daily activity profile of one or more comparable users; generating an adjusted user daily activity profile comprising adjusted activity features each comprising one or more of adjusted specific intensity and adjusted specific duration at intervals throughout at least a portion of a day to cumulatively provide the daily target activity level for the user; and outputting the adjusted user daily activity profile for use by the user.

One or more of the computer programs may, when run on a computer, cause the computer to configure any apparatus, including a battery, circuit, controller, or device disclosed herein or perform any method disclosed herein. One or more of the computer programs may be software implementations, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

One or more of the computer programs may be provided on a computer readable medium, which may be a physical computer readable medium such as a disc or a memory device, may be a non-transient medium, or may be embodied as a transient signal. Such a transient signal may be a network download, including an internet download.

The present disclosure includes one or more corresponding aspects, examples or features in isolation or in various combinations whether or not specifically stated (including claimed) in that combination or in isolation. Corresponding means (e.g. daily target activity level receiver, user daily activity profile receiver) for performing one or more of the discussed functions are also within the present disclosure.

The above summary is intended to be merely exemplary and non-limiting.

### Brief Description of the Figures

A description is now given, by way of example only, with reference to the accompanying drawings, in which:-
Figure 1 shows an example apparatus according to the present disclosure;
Figure 2 illustrates another example apparatus according to the present disclosure;
Figure 3 illustrates a further example apparatus according to the present disclosure;
Figure 4 illustrates schematically an example machine learning model according to the present disclosure;
Figure 5 illustrates schematically inputs to, and output from, an example apparatus according to the present disclosure;
Figure 6 illustrates schematically an example user daily activity profile according to the present disclosure;
Figures 7a-7d illustrate user daily activity profiles generated using a machine learning model for two different daily target activity levels according to the present disclosure;
Figures 8a-8d illustrate adjusted activity features according to the present disclosure;
Figures 9a-9b illustrate apparatus providing output of an adjusted daily activity profile according to the present disclosure;
Figure 10 shows the main steps of a method of using the present apparatus; and
Figure 11 shows a computer-readable medium comprising a computer program configured to perform, control or enable the method of Figures 9.

### Description of Specific Examples

Current developments in personal and lifestyle technology include devices and applications which can monitor user activity. For example, a user may wish to keep a record of where and how far they have travelled when out running. As another example, a user may wish to keep a record of their sleep patterns by monitoring their movements in bed. While it may be informative for a user to see a measure of their activity, such as a step count for the day, a user may wish to use personal monitoring technology to do more than monitor their current activities. For example, a user may wish to increase the distance they can comfortably run if training for a fun run, or may wish to increase the number of calories burned in a day to aid a fitness and weight loss programme. A user may wish to gradually decrease the amount of time spent exercising, for example to aid recovery from an injury, or increase the amount of time or number of topics when studying, for example in preparation for an exam.

It may be that a user will be better at achieving a change in their behaviour if they have a goal to aim for, rather than merely a non-specific desire to make the change. Having feedback in short or real-time cycles may also improve the chances of behaviour change compared with scenarios that do not include feedback or which include a greatly delayed feedback.

An extension to monitoring activity levels may be to use machine learning to learn from prior activity data and other information to predict the user's activity in the future (e.g. for the following day). Doing this may provide a way for a user, or a software developer to identify the time(s) at which a user is likely to carry out certain activities or have a certain activity level (e.g. a trip to the gym, walking home from work). A notification may be sent to the user encouraging them to take part in an activity just prior to when the activity is expected to begin, or reminding the user if they have missed the expected period of activity and suggesting an activity at another time to make up for the missed activity.

Providing reminders for a user regarding expected activities and activity levels may provide useful motivation for the user. Further incorporating a control system into the machine learning model may be done, to deliberately over-predict, or over-plan (or under-predict/under-plan if desired) upcoming activities in a way that still maintains consistency with the known data while providing a subtle change in activity compared with what the user has done previously. Small but manageable increases/decreases may be included in the activity predictions. The predictions could then be displayed/provided to the user with the intention that the user should strive to at least meet the prediction.

Since the prediction would be based on real data, its contours may match those likely to be made by the user if the user strives hard, rather than merely being a linear manipulation of the ordinary prediction. By using machine learning to understand the user's previous habits, and/or the habits of other users of a similar type to the user, then a prediction/aim may be generated which is a natural progression from the user's usual behaviour.

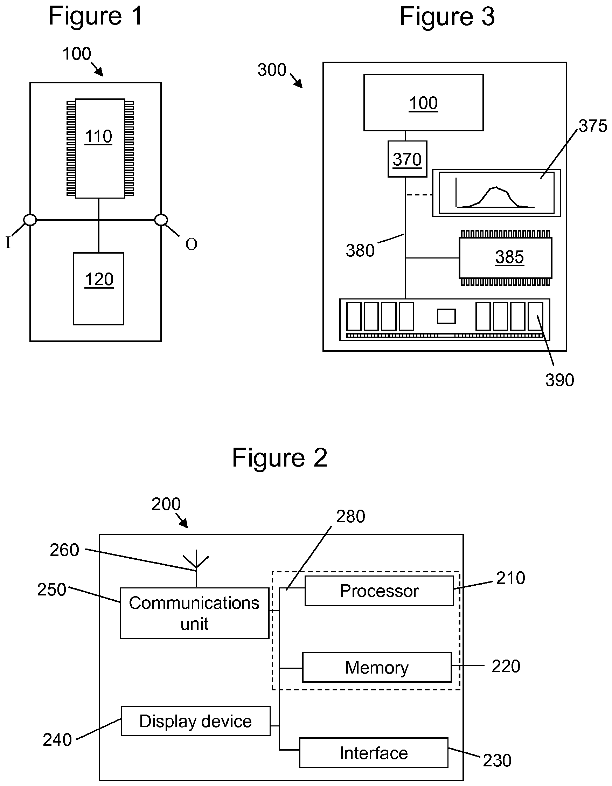

Figure 1 shows an apparatus 100 comprising a processor 110, memory 120, input I and output O. In this example only one processor and one memory are shown but it will be appreciated that other examples may utilise more than one processor and/or more than one memory (e.g. same or different processor/memory types). The apparatus 100 may be or may comprise an application specific integrated circuit (ASIC). The apparatus 100 may be or may comprise a field-programmable gate array (FPGA). The apparatus 100 may be a module for a device, or may be the device itself, wherein the processor 110 is a general purpose CPU and the memory 120 is general purpose memory.

The input I allows for receipt of signalling to the apparatus 100 from further components. The signalling may represent a daily target activity level for the user, or may represent a user daily activity profile, for example. The output O allows for onward provision of signalling from the apparatus 100 to further components. For example, an adjusted user daily activity profile may be output. In this example the input I and output O are part of a connection bus that allows for connection of the apparatus 100 to further components. The processor 110 is a general purpose processor dedicated to executing/processing information received via the input I in accordance with instructions stored in the form of computer program code on the memory 120. The output signalling generated by such operations from the processor 110 is provided onwards to further components via the output O.

The memory 120 (not necessarily a single memory unit) is a computer readable medium (such as solid state memory, a hard drive, ROM, RAM, Flash or other memory) that stores computer program code. This computer program code stores instructions that are executable by the processor 110, when the program code is run on the processor 110. The internal connections between the memory 120 and the processor 110 can be understood to provide active coupling between the processor 110 and the memory 120 to allow the processor 110 to access the computer program code stored on the memory 120.

In this example the input I, output O, processor 110 and memory 120 are electrically connected internally to allow for communication between the respective components I, O, 110, 120, which may be located proximate to one another as an ASIC. In this way the components I, O, 110, 120 may be integrated in a single chip/circuit for installation in an electronic device. In other examples, one or more or all of the components may be located separately (for example, throughout a portable electronic device such as devices 200, 300, or within a network such as a "cloud" and/or may provide/support other functionality).

One or more examples of the apparatus 100 can be used as a component for another apparatus as in Figure 2, which shows a variation of apparatus 100 incorporating the functionality of apparatus 100 over separate components. In other examples the device 200 may comprise apparatus 100 as a module (shown by the optional dashed line box) for a mobile phone or PDA or audio/video player or the like. Such a module, apparatus or device may just comprise a suitably configured memory and processor.

The example apparatus/device 200 comprises a display 240 such as, a Liquid Crystal Display (LCD), e-Ink, or touch-screen user interface (like a tablet PC). The device 200 is configured such that it may receive, include, and/or otherwise access data. For example, device 200 comprises a communications unit 250 (such as a receiver, transmitter, and/or transceiver), in communication with an antenna 260 for connection to a wireless network and/or a port (not shown). Device 200 comprises a memory 220 for storing data, which may be received via antenna 260 or user interface 230. The processor 210 may receive data from the user interface 230, from the memory 220, or from the communication unit 250. Data may be output to a user of device 200 via the display device 240, and/or any other output devices provided with apparatus. The processor 210 may also store the data for later user in the memory 220. The device contains components connected via communications bus 280.

The communications unit 250 can be, for example, a receiver, transmitter, and/or transceiver, that is in communication with an antenna 260 for connecting to a wireless network and/or a port (not shown) for accepting a physical connection to a network, such that data may be received via one or more types of network. The communications (or data) bus 280 may provide active coupling between the processor 210 and the memory (or storage medium) 220 to allow the processor 210 to access the computer program code stored on the memory 220.

The memory 220 comprises computer program code in the same way as the memory 120 of apparatus 100, but may also comprise other data. The processor 210 may receive data from the user interface 230, from the memory 220, or from the communication unit 250. Regardless of the origin of the data, these data may be outputted to a user of device 200 via the display device 240, and/or any other output devices provided with apparatus. The processor 210 may also store the data for later user in the memory 220.

Device/apparatus 300 shown in figure 3 may be an electronic device (including a tablet personal computer), a portable electronic device, a portable telecommunications device, a wearable electronic device, or a module for such a device. The apparatus 100 can be provided as a module for device 300, or even as a processor/memory for the device 300 or a processor/memory for a module for such a device 300. The device 300 comprises a processor 385 and a storage medium 390, which are electrically connected by a data bus 380. This data bus 380 can provide an active coupling between the processor 385 and the storage medium 390 to allow the processor 380 to access the computer program code.

The apparatus 100 in figure 3 is electrically connected to an input/output interface 370 that receives the output from the apparatus 100 and transmits this to the device 300 via data bus 380. Interface 370 can be connected via the data bus 380 to a display 375 (touch-sensitive or otherwise) that provides information from the apparatus 100 to a user. Display 375 can be part of the device 300 or can be separate. The device 300 also comprises a processor 385 that is configured for general control of the apparatus 100 as well as the device 300 by providing signalling to, and receiving signalling from, other device components to manage their operation.

The storage medium 390 is configured to store computer code configured to perform, control or enable the operation of the apparatus 100. The storage medium 390 may be configured to store settings for the other device components. The processor 385 may access the storage medium 390 to retrieve the component settings in order to manage the operation of the other device components. The storage medium 390 may be a temporary storage medium such as a volatile random access memory. The storage medium 390 may also be a permanent storage medium such as a hard disk drive, a flash memory, or a nonvolatile random access memory. The storage medium 390 could be composed of different combinations of the same or different memory types.

Figure 4 illustrates schematically an example machine learning model 400 according to the present disclosure. Using a machine learning technique, for example a deep learning and generative method such as a Variational Autoencoder (VAE) or Deep Conditional Generative Adversarial Network (DCGAN), it is possible to produce predictions about the activity of a user for the following day in an output predicted activity 412. These predictions may be based on an input 402 containing the activity information of the user from the previous day, as well as other conditioning data (e.g. the time of day 414, and/or demographic and psychometric data about the user 416).

The input activity 402 provided to the machine learning model is the training data used to teach the machine learning model what the user's activity habits and trends are. The input activity 402 may comprise at least one previous user daily activity profile of the user. For example, the activity of a user may have been monitored and recorded for one or more previous days. Examples include a user recording their walking and running using a pedometer, a user recording their movements and breathing patterns during sleep, or a user keeping a diary-style record of their daily activities in an activity logging application.

Instead or as well, the input activity 402 may comprise at least one previous user daily activity profile of one or more comparable users. For example, the user may not have already recorded one or more user daily activity profiles, or the user may only have recorded one or two previous user daily activity profiles, and the previous user daily activity profile of one or more comparable users may be used to supplement the user's personal activity data with data from similar users.

A comparable user may be a user having one or more matching demographic factors with the user, such as age (e.g. 25 years old), age range (e.g. 25-24 years old), gender, weight (e.g. 76kg, 65-75kg), activity level (e.g. a user may be classed as a "low activity" user if they do no exercise and work in an office based job, or a user may be classed as a "high activity" user if they have a manual labour job and visit the gym most evenings), daily routine (e.g. a user who exercises in the morning, rests until 6pm then exercises for an hour in the evening), activity type (e.g. the comparable user and the user both go horse-riding), profession (e.g. a builder may have a different working activity level and daily schedule than an accountant), income, geographical location, ethnicity and disability.

The input activity 402, that is, the prior data which is applicable to the user, provides a user daily activity profile, and, along with one or more other factors 414, 416, 418 (described below) which are received by an apparatus, is used to generate an adjusted user daily profile 412. The machine learning model 400 may comprise a generative layer 404 for generating the adjusted user daily profile 412.

Another factor to be accounted for in generating the adjusted user daily profile 412 is a time conditioning factor 414. This factor 414 may be a binary vector (for example, a one-hot encoded binary vector) time data entry. A one-hot vector is a vector filled with zeros except for a single 1 value somewhere. The position of the 1 indicates, for example, the time of day or day of the week. It may be easier to train deep learning models using such vectors rather than using unencoded times of day; however, it is still possible to train them without using the vector encoding. The time conditioning factor 414 is received by the apparatus and used to generate the adjusted user daily profile 412. The time conditioning factor 414 may, for example, indicate a particular time of day of importance to consider in generating the adjusted user daily profile 412, such as a mealtime or a period during the night where the user is unlikely to be active, or a period in the daytime which provides a good opportunity for the user to perform an activity. The machine learning model 400 may comprise a time conditioning module 406 for handling time data for use in generating the adjusted user daily profile 412.

Another factor to be accounted for is a global conditioning factor 416, which may be used to generate an adjusted user daily profile 412 by indicating one or more demographic descriptors of the user. Any demographic descriptor may be coded for use by the apparatus, such as the user's age, gender, geographic location, or other demographic. The global conditioning factor 416 may be used in examples where the input activity 402 comprises at least one previous user daily activity profile of one or more comparable users; that is one or more users having at least one common demographic descriptor with the user. For example, only data from other female users may be used to train the machine learning model for use by a female user. As another example, data from other users of the same age, sex, height, weight and profession as the user may be used to train the machine learning model. The machine learning model 400 may comprise a global conditioning module 408 for handling data from other users having matching demographic factors for use in generating the adjusted user daily profile 412.

Another factor to be accounted for is a control variable/value 418, which may be called a daily target activity level for a user which is received by the apparatus and used to generate an adjusted user daily profile 412. The machine learning model 400 may comprise a control variable module 410 for including the daily target activity level 418 in the modelling used to generate the adjusted user daily profile 412.

By including the control value 418 piece of conditioning data additionally to the input activity 402, it is possible to train the model 400 to learn not only to make a prediction about the user activity of the following day 412, but also to simultaneously learn the relationship between the input control value 418 and the output overall activity level (which may be termed the particular activity level for the day). The particular activity level for the day is provided cumulatively by the activities performed throughout the day as recorded in the received user daily activity profile for the user 402.

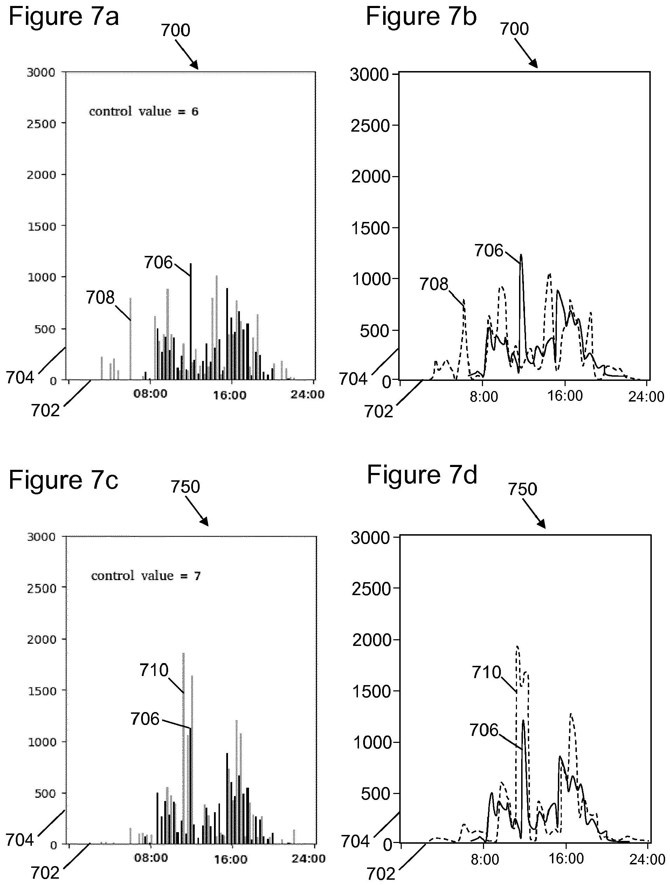

Training the model 400 using the control value 418 can be done by two methods. Both methods were used in experiments which provided the data shown in Figures 7a-7d. In a first method, in the training data 402, the control value 418 is always set to be a value corresponding to the sum of the activity data for the following day. In experiments the control value was set to a number between 0 and 9, normalised according to range of all activity sums in the training data (thus preventing unseen control values at testing time). This allowed the machine learning model 400 to learn that there is a simple correlation between this control value 418 and sum of the activity it should predict at the output 412.

In a second method, to further force the machine learning model 400 to respect this correlation, an additional loss term can be introduced into the objective function when training. The objective function, sometimes called the 'cost function' or 'loss function', is the function that the machine learning model uses to identify how much error there is in a particular output/prediction. As the machine learning model is training, it will attempt to minimise the error of its outputs, as defined by the objective function. Therefore an objective function that is defined as the difference between the predicted activity profile and the actual activity profile will encourage the machine learning model to minimise the error in the predictions of the activity profile.

In some examples an objective function may be used which comprises three terms: a reproduction loss, a generative loss, and a novel loss. The reproduction loss (which may be the mean squared error of the output activity profile (adjusted user daily activity profile) compared with the actual activity profile that later occurred) encourages the model to learn to reproduce activity profiles with the correct values (i.e. to predict adjusted user daily activity profiles 412 which match the user daily activity profile 402 used in training, adjusted according to the daily target activity level (the control value 418)).

The generative loss (which may be formulated in various ways, such as using a Kullback-Leibler divergence which allows for a type of generative model known as a Variational Auto-Encoder, but other options are available) allows the model to work as a generative model. Thus, it is possible to run the model following training without using at least one previous user daily activity profile of the user or a comparable user as input, and providing only a control value (the daily target activity level) as input. In this way, it is possible for a user to use the model even if no data exists for that particular user, because the model has been trained using data from one or more comparable users (e.g. from a demographic matching that of the user) and the generative loss term allows the model to be run without requiring a user daily activity profile as input.

The novel loss term, which may be called the control-variable loss, may be formulated as the mean squared error between the sum of the predicted activity (normalised to the scale of the control value) and the control value. The novel loss term encourages the model to produce an output adjusted user daily activity profile with an overall activity level corresponding to the control variable input (the daily target activity level). These three loss terms may be weighted according to importance, giving the model relatively more or less incentive to achieve particular goals.

The mean squared error between the sum of the predicted activity (normalised to the scale of the control value) and the control value 418 was used in experiments as shown in Figures 7a-7d. Thus the machine learning model 400 has an incentive to minimize both the error in its prediction 412 overall, as well as a separate incentive (which can be weighted as required) to minimise the error in the sum of the predicted activity.

After training the machine learning model with at least the input activity 402 and a control variable 418, an adjusted user daily profile 412 may be generated and output for use by the user. It is possible, using the generative loss term, to provide the control variable 418 only as an input without also providing any user daily activity profile for the user (because the model has been trained using data from one or more other comparable users), but the output may be fairly smooth and generic (e.g. an average over the training data from the comparable user(s)) compared with the output obtained from providing the input activity profile for the user as well. That is, after training the model is complete, it may not be necessary to provide any input at all to the model other than a daily target activity level (control variable 418) for the user. It may be that the more types of input that are provided to the model, the more accurate the output will be.

In some examples, it may be possible to omit the use of one or more previous user daily activity profiles from the user by using latent variables in the generative layer 404, and/or by using dropout and binary switches. Firstly, some latent values may be inserted in the generative layer 404 for sampling. These values may be randomly selected, or picked from values previously assigned to other users in the training data (which results in a generated profile that behaves how the user would be expected to behave). Secondly, for every control value 418, a secondary binary value may be provided as an input that acts like a switch. In training, the binary value may be set to 0 or to 1, at random. Whenever the binary value is set to 0, the control value 418 is also set to 0. In this way, the model 400 learns that when the binary value is 0, the control value 418 is meaningless. These secondary binary values act as switches that indicate whether or not the control values 418 are present. By setting up the model 400 in this way, it may be possible to generate an adjusted user daily activity profile even if there is "missing" input data (e.g. no data for a particular week of the year), and it may be possible to generate an adjusted user daily activity profile at time t=0, that is, for a new user with no existing activity data. Thus, even if nothing is known about the activity of a particular new user, a general prediction may still be made to obtain an adjusted user daily activity profile for the first day/time interval for that user.

The apparatus may be configured to output adjusted user daily activity profiles for a new user without basing the user daily input profile (in training) on any user daily activity profile of the user (because little or no such data exists), until a predetermined amount of data is recorded for that user, at which point the apparatus may be configured to include at least one previous user daily activity profile of the user in obtaining the user daily activity profile (e,g. to provide an adjusted user daily activity profile which is more personally tailored to the actual user's activity habits).

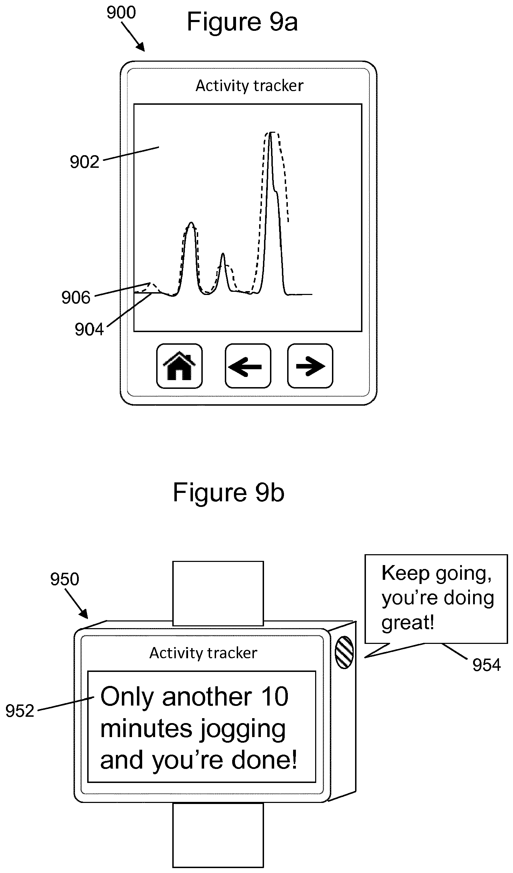

Examples of output direct to the user are discussed in relation to Figures 9a-9c. In some examples, the output may be output to a developer system which can use the data, for example, to train other machine learning models for the current user, and/or for other users, or to further process the output adjusted user daily activity profile for onward provision to the user. For example, the adjusted user daily profile 412 may be output to a developer system which has access to other details of the user, such as the user's name or personal user preferences, so that the adjusted user daily activity profile can be personalised before being provided to the user by the developer system.

In production mode (after the model 400 is trained) the control value 418 can be set by the developer and/or the user. For example, if previous activity data 402 for the user usually corresponds to a control value (a particular activity level for the day) of 4, then the developer and/or the user can set a control value (a received daily target activity level for the user) 418 of 4.5 when making an activity prediction. Doing this will result in a predicted activity profile 412 that is not merely slightly higher than otherwise, but actually has peaks and troughs in the places that are appropriate for such an increased level of activity. This is shown in the experimental data presented in Figures 7a-7d described later.

Figure 4 may be considered to illustrate a machine learning model 400 which an apparatus 100, 200, 300 is configured to use. The machine learning model 400 is configured to identify one or more of the activity features in the user daily activity profile 402 as candidates for adjustment based on one or more of at least one previous user daily activity profile of the user 402; and at least one previous user daily activity profile of one or more comparable users 416; and adjust the one or more identified candidate activity features, in accordance with the daily target activity level for the user 418, to generate the adjusted user daily activity profile 412.

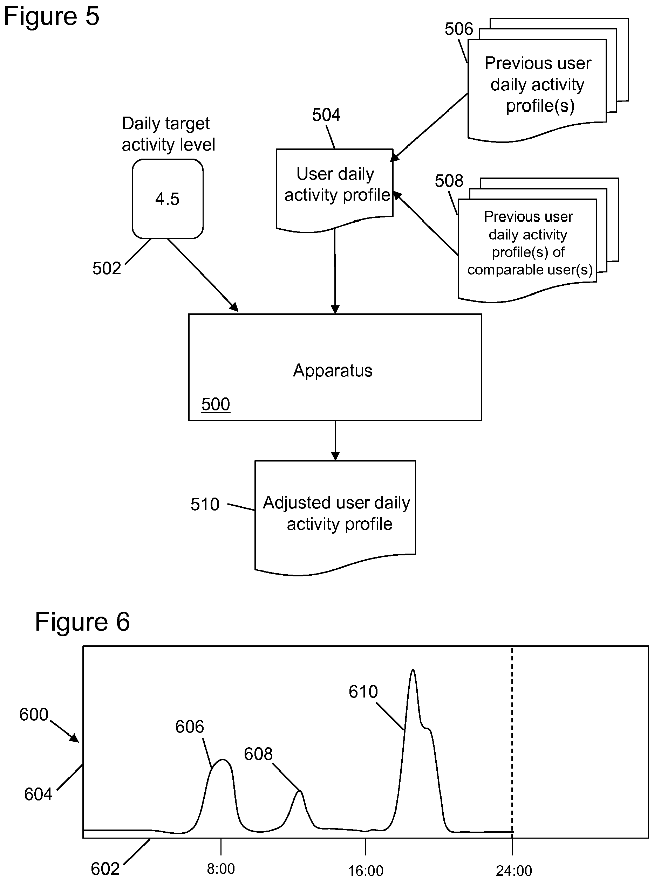

Figure 5 illustrates schematically inputs to, and output from, an example apparatus according to the present disclosure. The apparatus 500 receives, as input, the daily target activity level for a user 502 (i.e. a control value used to adjust the input activity profile) and a user daily activity profile for the user 504 (e.g. the activity profile of the user from the previous day). The user daily activity profile 504 comprises activity features each comprising specific intensity and specific duration at intervals throughout at least a portion of a day, as described in more detail with reference to Figure 6. The user daily activity profile 504 cumulatively provides a particular activity level for the day. For example, the area under the activity profile curve may be determined to provide the particular activity level for the day, and normalised for comparison with the daily target activity level 502.

The user daily activity profile 504 is based on one or more of: at least one previous user daily activity profile of the user 506; and at least one previous user daily activity profile of one or more comparable users 508. Figure 5 illustrates the at least one previous user daily activity profile of the user 506 and at least one previous user daily activity profile of one or more comparable users 508 leading to the generation of the user daily activity profile 504 without the apparatus 500 being involved at this stage. It should be understood that, in other examples, the at least one previous user daily activity profile of the user 506 and at least one previous user daily activity profile of one or more comparable users 508 may be illustrated as being input to the apparatus 500 for the apparatus 500 to generate the user daily activity profile 504 (for example, if no user data is yet available, an input user daily activity profile 504 may be generated from data from other users having matching demographics to the current user 508, as a starting point for generating an adjusted user daily activity profile 510 for the user). In some examples, previous user daily activity profiles 506 may be used to obtain the user daily input profile 504, while previous user daily activity profiles of comparable users 508 may be used by the apparatus 500 to train a machine learning model, so that the apparatus 500 can generate the adjusted user daily activity profile 510 by adjusting the user's personal activity profile inputs 504 in a way which is consistent with real-world activity of other comparable users 508 and in agreement with the daily target activity level 502.

The apparatus 500 is configured to generate an adjusted user daily activity profile 510 comprising adjusted activity features each comprising one or more of adjusted specific intensity and adjusted specific duration at intervals throughout at least a portion of a day to cumulatively provide the daily target activity level for the user 502; and output the adjusted user daily activity profile 510 for use by the user.

Figure 6 illustrates schematically an example user daily activity profile 600 according to the present disclosure. This simplified user daily activity profile is shown as activity level 604 on the y-axis plotted against time 602 on the x-axis. Activity level 604 as shown in Figure 6 may be a plot of for example, heart rate or body temperature, which are parameters indicating the activity of a user. A resting level is represented at the minima of the curve, and an increase from the resting level is shown due to activities which raise the heart rate or body temperature. As plotted in Figure 6, the activity level 604 may also indicate the differential of, for example, calories used, steps taken, distance travelled, completion status of a task, or time asleep, which are all parameters which accumulate over the period of a day. Of course, the activity level for a user may alternatively be plotted as a cumulative step-type plot of calories used, steps taken, distance travelled, completion status of a task, or time asleep in some examples.

For example, as shown in Figure 6, the user may have gone for a morning jog 606 around 8am as a medium intensity activity, then rested until lunchtime when they went for a low intensity walk 608. At around 19:00 the user went to the gym and did a high intensity (e.g. cardio) exercise session followed by a lower intensity (e.g. swimming) activity 610.

Figures 7a-d illustrate the effect of setting a control value (a received daily target activity level for the user) 418 of "6" (Figures 7a and 7b), and "7" (Figures 7c and 7d), compared with the lower particular activity level for the day (for example, the lower particular activity level for the day was "5"). Figures 7a and 7b illustrate the same data, in Figure 7a as a histogram and in Figure 7b as a line graph, for a received daily target activity level for the user of 6. Figures 7c and 7d illustrate the same data, in Figure 7c as a histogram and in Figure 7d as a line graph, for a received daily target activity level for the user of 7. Activity intensity is plotted on the y-axis 704 against time of day on the x-axis 702.

The daily target activity level for the user may indicate an increase or decrease in activity level compared to a particular activity level of at least one previous user daily activity profile of the user (for example, if the user usually records how many steps they have taken during the day then the user's past recorded step activity profiles may be used as a basis to generate the adjusted activity profile). In the examples of Figures 7a-7d it indicates an increase in activity level. Instead, or as well, the daily target activity level for the user may indicate an increase or decrease in activity level compared to a particular activity level of at least one previous user daily activity profile of one or more comparable users (for example, if the user is new to exercise, then activity profiles from other users of a similar demographic may be used as a starting point to generate the adjusted activity profile).

The same user daily activity profile 706 for the user is shown on all of Figures 7a-7d. The predicted/modelled user daily activity profile 708 for a control value of 6 is shown in Figures 7a - 7b, and it can be seen that additional features are present early in the day which represent activity that the user could perform at those times to achieve the higher overall activity level for the day. The adjusted user daily activity profile 708 in this example comprises an adjusted activity feature at a particular time of day (early morning) corresponding to a time of no activity in the user daily activity profile 706.

The higher control value of 7 shown in Figures 7c and 7d does not merely result in the same predictions with every bar slightly higher than if the control value is 6, as shown in Figures 7a and 7b, but in fact results in a different overall shape 710. For example, there are some small additional features early in the day but no higher intensity feature as shown in figures 7a-7b. A high intensity feature is present in the predicted/modelled user daily activity profile 710 around noon which is a higher intensity peak than the medium intensity peak at that time in the user daily activity profile 706. The change in user daily activity profile shape with different control number corresponds to the fact that when users are more active they do not merely, for example, take more steps consistently throughout the day, but can undergo periods of more intense activity (e.g. going to gym or taking a fast walk).

The apparatus may be configured to generate the adjusted user daily activity profile 708, 710 by adjusting activity features in the user daily activity profile 706 to minimise the perceived change in daily activity by the user whilst still achieving the daily target activity level for the user. For example, by introducing a low intensity short duration new activity feature at a time when the user may not usually be active, the user may benefit from increased activity levels by being encouraged to perform a relatively easy activity in their spare time. This example may help break a user's habit of not doing any activity during a period of the day when they didn't realise that they did have the time to do an activity. As another example, if a user usually goes for a jog around the same route each night, then the user may be encouraged to jog a little further, or take a slightly different route (which requires slightly more effort) during their usual "jogging" activity time. The user may therefore benefit by increasing their activity level without feeling like a big new effort has been required (because they would have been jogging anyway) but also benefit from a break in their usual routine, to keep the jogging activity fresh and interesting.

In other examples, the apparatus may be configured to generate the adjusted user daily activity profile by adjusting one or more of the specific intensity and specific duration of the activity features of the user daily activity profile evenly over the at least a portion of a day, in accordance with the daily target activity level for the user, to provide an adjusted user daily activity profile. For example, an adjusted user daily activity profile may be the user daily activity profile with an even 10% increase in intensity over all features, or may be the user daily activity profile with each activity feature extended in time by a further 10% duration for that activity. Evenly adjusting the intensity, or duration, to obtain the adjusted user daily activity profile may be useful, for example, if the daily activity profile relates to a baby's sleep pattern, and the parent wants to try and decrease the length of time the baby sleeps during the day to encourage a longer uninterrupted sleep at night time. In this case each "sleep" activity feature during the day may be reduced by 10% in duration, so the parent is notified to wake the baby a little earlier than usual after daytime sleeps. The apparatus may be configured to play a song at "wake-up time" according to the adjusted user daily activity profile to help gently wake the baby.

The user daily activity profiles 708, 710, which are adjusted versions of the received user daily activity profile 706, are generated by the apparatus and output for use by the user. By showing the user their predicted activity 708, 710 in advance (e.g. at the start of the day) and then challenging the user to meet (or exceed) it, the user may be encouraged to be more active than they would otherwise have been, and can check in real-time how they are faring against their prediction.

There are different ways to set the control value over time. Simply not setting the control value, or setting it to be equal to the particular activity level for the user for a previous day (or days), would result in an accurate current activity prediction (without any over-predicting to increase the activity level for the user for the day, or under-predicting to decrease the activity level for the user for the day). If the user wanted to increase the level of activity they were performing they can set a higher control value (daily target activity level) than the activity level for one or more previous days, and the apparatus can generate an adjusted user daily activity profile comprising adjusted activity features which, if followed by the user, would raise their activity level for the day. The user may wish to do this if they are training for a big sports event, or wish to improve their overall fitness levels. If the user wanted to decrease the level of activity they were performing they can set a lower control value (daily target activity level) than the activity level for one or more previous days, and the apparatus can generate an adjusted user daily activity profile comprising adjusted activity features which, if followed by the user, would lower their activity level for the day. The user may wish to do this if they have just competed in a big sports event, and wish to relax in a controlled way to maintain their fitness but avoid burning out now the event has taken place.

In other examples the user could be placed on a schedule of gradually increasing control value/daily target activity level over a predetermined time period. For example, this may be implemented using a reinforcement learning system so that the apparatus "learns" (e.g. by using a machine learning model) the optimal value to set the control value to encourage the user gradually to change their activity. For example, if the user easily reaches the target activity value then the target value may be changed further for the following day; if the user is struggling to meet the target activity value then the target value may be changed to be closer to the activity value for the current day. In some examples the apparatus may be said to be configured to generate a series of adjusted user daily activity profiles which progressively provide an intermediate daily target activity level for the user over a predetermined plurality of days. The intermediate daily target activity level for the user varies progressively over the predetermined plurality of days until it equals the daily target activity level for the user.

In other examples, the apparatus may be configured to set the control value/daily target activity level according to a heuristic derived from clinical or social science experiments that demonstrate good strategies to encourage behaviour change. For example, studies may show that for women aged 34-45, early morning is the best time of day to exercise and burn body fat. Thus, for a 40 year old woman who wishes to lose weight, the apparatus may provide an adjusted user daily activity profile which includes increased levels of activity in the early morning.

In some examples, there may be feedback to adjust the adjusted user daily activity profile according to whether or not the daily target activity level has been met or not. Dependent on the user daily activity profile for the user for a particular day, the apparatus may be configured to adjust the daily target activity level for the user for a subsequent day to account for user activity recorded on the particular day. If the particular activity level for the particular day is higher than daily target activity level, the apparatus may be configured to increase the daily target activity level for the subsequent day. For example, if the user is reaching (or possibly exceeding) their activity goals by achieving a higher activity level than the daily target activity level, then the daily target activity level may be increased for the following day (e.g. up to a predetermined maximum level, for example for safe exercising).

If the particular activity level of a user for the particular day is lower than daily target activity level, the apparatus may be configured to decrease the daily target activity level for the subsequent day. For example, if the user has not been studying at all and decided to aim to study for two hours a day, then is only managing one and a half hours a day, the daily target activity level may be adjusted from two hours to one and a half hours to give the user a more manageable goal. The one-and-a-half-hour goal, once met, may then be incrementally increased over a predetermined period of several days (e.g. a week) to the original two hours target for study activity.

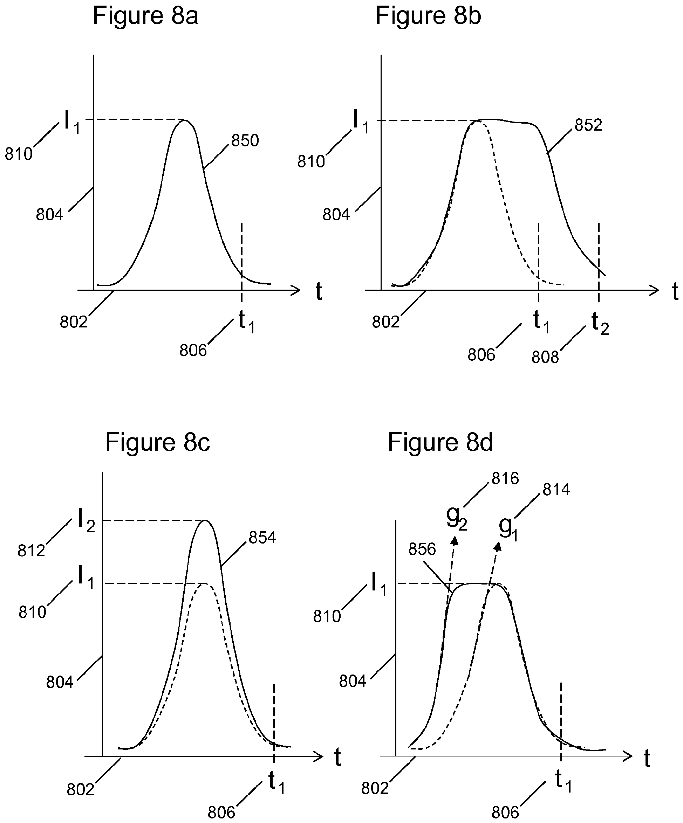

Figures 8a-8d illustrate adjusted activity features according to the present disclosure. The intensity level of activity is plotted on the y-axis 804 against time plotted on the x-axis 802. Figure 8a shows an activity peak 850 (for example, a user goes for a bike ride). The activity has a duration of time t₁ 806 (e.g. one hour), and a peak intensity of I₁ 810 (e.g. a heart rate of 150 bpm).

Figure 8b shows the original activity peak 850 as a dashed line compared with an adjusted activity feature 852 of an adjusted user daily activity profile. The adjusted activity feature 852 comprises the same specific intensity I₁ 810 but an adjusted specific duration t₂ 808 (e.g. one hour and 30 minutes) compared to the corresponding activity feature 850 in the user daily activity profile. The adjusted activity feature 852 indicates that the user can work just as hard as they did before according to the activity feature 850 in the user daily activity profile, but carry out the activity for longer, to increase their overall activity. For example, the user may go on a longer bike ride at the same pace as before.

In some examples, the apparatus may be configured to provide a suggested activity to meet the requirements of the adjusted activity feature. For example, if the user tracks their bike rides using GPS technology, the apparatus may be configured to determine a longer route than the route usually taken (as recorded by the GPS technology), and show the new adjusted route to the user, wherein the longer route would provide the user with an adjusted duration (and/or intensity) matching the adjusted activity feature 852. Another example relating to adjusted specific duration of an activity is the apparatus suggesting that the user completes 12 revision questions in preparation for an examination rather than the 10 usual questions (the user may log which questions they have attempted, and the apparatus has access to the logged data, for example).

Figure 8c shows the original activity peak 850 as a dashed line compared with an adjusted activity feature 854 of an adjusted user daily activity profile. The adjusted activity feature 854 comprises the same specific duration t₁ 806 but an adjusted specific intensity I₂ 812 (e.g. a heart rate of 160 bpm) compared to the corresponding activity feature 850 in the user daily activity profile. The adjusted activity feature 854 indicates that the user can work harder than they did before, but for the same period of time, according to the activity feature 850 in the user daily activity profile, to increase their overall activity. For example, the user may go on a bike ride of a similar distance as before, but which takes a different route involving steeper climbs. Another example relating to adjusted specific intensity of an activity is the apparatus suggesting that the user takes an hour revising a subject for an exam as before, but chooses more difficult questions than previously attempted, thereby increasing the "intensity" of the activity. As another example, a user may attend a step aerobics class and usually have their step board a height of two blocks from the ground. For the higher intensity adjusted activity the user may be prompted to have the step board three blocks off the ground for the same class, to increase how hard the user has to exercise to perform the class.

Figure 8d shows the original activity peak 850 as a dashed line compared with an adjusted activity feature 856 of an adjusted user daily activity profile. The adjusted activity feature 856 comprises the same specific duration t₁ 806 and same specific intensity I₁ 810 compared to the corresponding activity feature 850 in the user daily activity profile, but has an adjusted gradient g₂ 816 between minimum and maximum 810 intensity levels of the adjusted activity feature 856 compared to gradient g₁ 814 of the corresponding activity feature 850 in the user daily activity profile. While Figure 8d shows an adjusted gradient at the start of the activity, there may be an adjusted gradient at the end of the activity instead, or as well, in some examples. The adjusted activity feature 856 indicates that the user can work at the same intensity and for the same period of time as before, but they should aim to "get up to speed", i.e. achieve the maximum intensity I₁ 810 more quickly than before. For example, a user may swim and find front crawl more intensive than breaststroke. The user may usually swim 10 lengths of breaststroke followed by 10 lengths of front crawl in their user daily activity profile. The adjusted activity profile for the user may be achieved by the user swimming 4 lengths of (lower intensity) breaststroke followed by 16 lengths of (higher intensity) front crawl to achieve the higher gradient g₂ 816.

While variations in duration, intensity, and gradient have been illustrated separately in Figures 8a-8d it will be appreciated that two or more factors may be combined in a single adjusted activity feature in an adjusted user daily activity profile, and may decrease rather than increase in some examples.

There may be a database listing possible activities which are within the range suitable for a particular user. Such a database may be stored on a remote server (remote from the apparatus) or in the "cloud". Activities listed in the database within the user's range may be, for example, within the fitness range of the user (e.g. casual, medium, active, competitive sports), within the geographical range of the user (e.g. cycle or jogging routes close to the user's house or work, classes held at the user's local gym), or within the scope of activities of interest to the user as determined from user-entered preferences or previously recorded user activities.

The apparatus may be configured to determine, from such a database, one or more suitable activities matching the adjusted activity level of an adjusted user daily activity profile, and suggest at least one of these suitable activities to the user. For example, to achieve a particular adjusted activity feature (e.g. have a heart rate of 140 bpm for 15 minutes, or undergo medium-intensity activities for half an hour), the apparatus may look up matching activities from the database which match the user's current location, time of day, and/or personal preferences, and suggest them to the user (e.g. attend a kickboxing class starting in 30 minutes at Joe's gym, or take a bike ride around a route presented on a map to the user close to the user's place of work). The apparatus may have access to a user's calendar to determine one or more suitable activity times (e.g. activities during the user's working hours would not be suggested).

Figures 8a-8d show an activity feature 850 compared with an adjusted activity feature 852, 854, 856. The two features compared in each of Figures 8a-8d may be considered to correspond to each other, because the adjusted activity feature is an adjusted version of the activity feature. An adjusted activity feature may correspond to an activity feature by each feature occurring at the same time of day (e.g. the user goes to step class between 6pm and 7pm each Wednesday). An adjusted activity feature may correspond to an activity feature by each relating to the same activity type (e.g. a user may go cycling for an hour, but it may be in the morning, or in the evening. No matter what time the user does the activity, it is the same activity type). An adjusted activity feature may correspond to an activity feature by the adjusted activity feature providing the same percentage contribution to the daily target activity level for the user as the activity feature provides to the particular activity level of the user daily activity profile. For example, a user may set a daily target activity level of burning 500 calories per day. If a user can walk in the morning for an hour to burn 100 calories, or jog in the evening for half an hour to burn 100 calories, then these two activities may be considered to correspond because they have the same calorie-burning contribution to the user's daily target activity level. In some examples, an activity feature may correspond to an adjusted activity feature in two or more such ways.

In some examples, the apparatus may obtain data (e.g. from a user's calendar) that the user has particular appointments during particular days which limit the adjustments which may be made to the user daily activity profile to obtain an adjusted user daily activity profile. For example, if a user is at work from 9am until 4pm, then the apparatus may avoid setting activities during this period because the user is not available to perform them. As another example, if a user usually cycles to work between 8:30am and 9:00am, the apparatus may not adjust this cycling activity so that it finishes at a later time, because the user would then be late for work. The apparatus may, however, suggest the user sets off 10 minutes earlier and takes an alternative cycling route to work, thereby slightly adjusting the user's activity level in relation to this morning cycling activity, but in a way which fits in with the user's schedule. Thus, the apparatus may be configured to generate an adjusted user daily activity profile in accordance with one or more fixed time constraints during the day.

Figures 9a-9b illustrate apparatus 900, 950 providing output of an adjusted daily activity profile 906, 952 according to the present disclosure. In Figure 9a, the user has an apparatus 900 which is a smartphone with a display screen 902. This apparatus is configured to output the adjusted user daily activity profile 904 for use by the user by providing for display of a graph indicating a current user daily activity profile 906 with the adjusted user daily activity profile 904 as a comparison. The user can then easily see how their current daily activity 906 compares with the adjusted user daily activity profile 904. The plotted current daily activity profile 906 may be updated in real time or periodically throughout the day.

In Figure 9b, the user has an apparatus 950 which is a smartwatch. The apparatus is configured to output the adjusted user daily activity profile for use by the user by providing a visual message 952 prompting the user to subsequently keep performing an upcoming activity (jogging) in the adjusted user daily activity profile. In this example the apparatus is also configured to providing an audio message 954 prompting the user to subsequently keep performing an upcoming activity in the adjusted user daily activity profile.

In other examples the apparatus may be configured to output the adjusted user daily activity profile for use by the user by providing a visual and/or audio message and/or haptic feedback prompting the user to perform an upcoming activity in the adjusted user daily activity profile. For example, the apparatus 950 may display a message indicating a future upcoming activity that the user could perform to achieve the adjusted user daily activity profile, such as "only half an hour to go until your gym session", "go to bed in 20 minutes" (if the user's activity is sleep and the user wishes to achieve a sleep goal), or "Hockey club meeting: 7pm tonight". The message need not necessarily specify the particular activity (e.g. "jogging" in Figure 9b) but may simply be an encouragement 954 to the user to continue training for a certain period of time to provide an activity feature matching the adjusted activity feature generated by the apparatus.

While daily activity profiles have been discussed, the apparatus may be configured to generate and output adjusted user daily activity profiles which provide adjustments to a user's activity profile over a longer time period. For example, if a user works from Monday to Friday (and thus has constraints on how and when activities are possible on those days), but is generally free on Saturdays and Sundays, then the apparatus may generate different adjusted user daily activity profiles on Mondays to Fridays than on Saturdays and Sundays, which may be adjusted to different extents depending on the day, in order to reach a weekly target activity level for the user. For example, if a user wants to increase her weekly number of calories burned, then a greater proportional increase in activities to burn calories may be included by the apparatus in the weekend adjusted user daily activity profiles than in the weekday adjusted user daily activity profiles. Of course, other cycles such as fortnightly, monthly, or longer periods, may be accounted for. A day may be considered to provide a period of time within which a user has enough time to aim to achieve a daily target activity level and obtain feedback in a timely enough way to show if they are succeeding or not, so that the user can try harder, or ease off, as required to meet the adjusted activity profile and daily target activity level.

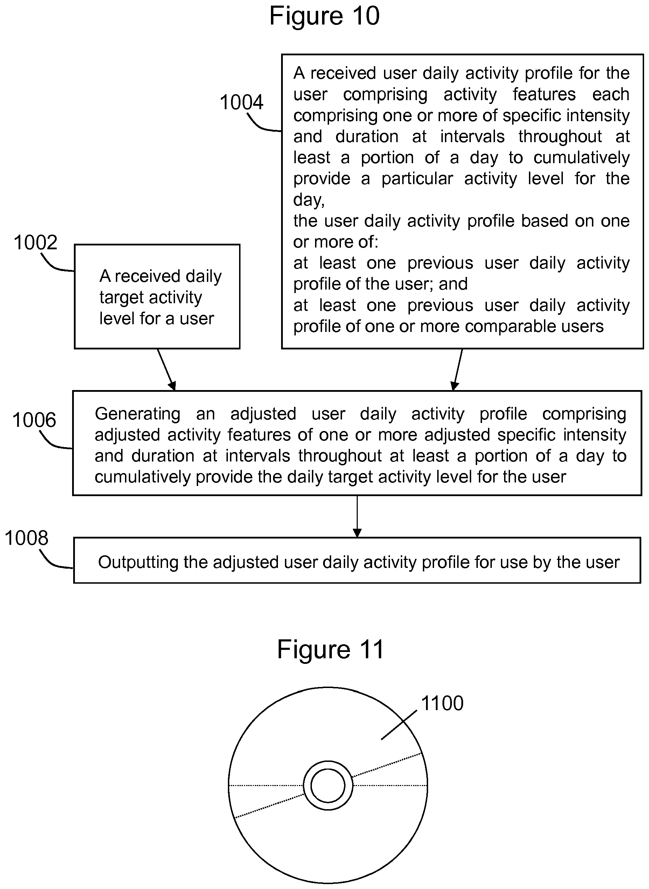

Figure 10 shows the main steps of a method of using the present apparatus. The method comprises, based on a received daily target activity level for a user 1002; and a received user daily activity profile for the user, the user daily activity profile comprising activity features each comprising specific intensity and specific duration at intervals throughout at least a portion of a day to cumulatively provide a particular activity level for the day, the user daily activity profile based on one or more of at least one previous user daily activity profile of the user; and at least one previous user daily activity profile of one or more comparable users 1004; generating an adjusted user daily activity profile comprising adjusted activity features each comprising one or more of adjusted specific intensity and adjusted specific duration at intervals throughout at least a portion of a day to cumulatively provide the daily target activity level for the user 1006; and outputting the adjusted user daily activity profile for use by the user 1008.

Figure 11 shows an example computer-readable medium comprising a computer program configured to perform, control or enable the method of Figure 10 or any method described herein. The computer program may comprise computer code configured to perform the method(s). In this example, the computer/processor readable medium 1100 is a disc such as a digital versatile disc (DVD) or a compact disc (CD). In other examples, the computer/processor readable medium 1100 may be any medium that has been programmed in such a way as to carry out an inventive function. The computer/processor readable medium 1000 may be a removable memory device such as a memory stick or memory card (SD, mini SD, micro SD or nano SD card).

It will be appreciated to the skilled reader that any mentioned apparatus/device and/or other features of particular mentioned apparatus/device may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/functional units.

In some examples, a particular mentioned apparatus/device may be pre-programmed with the appropriate software to carry out desired operations, and wherein the appropriate software can be enabled for use by a user downloading a "key", for example, to unlock/enable the software and its associated functionality. Advantages associated with such examples can include a reduced requirement to download data when further functionality is required for a device, and this can be useful in examples where a device is perceived to have sufficient capacity to store such pre-programmed software for functionality that may not be enabled by a user.

It will be appreciated that any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

It will be appreciated that any "computer" described herein can comprise a collection of one or more individual processors/processing elements that may or may not be located on the same circuit board, or the same region/position of a circuit board or even the same device. In some examples one or more of any mentioned processors may be distributed over a plurality of devices. The same or different processor/processing elements may perform one or more functions described herein.

It will be appreciated that the term "signalling" may refer to one or more signals transmitted as a series of transmitted and/or received signals. The series of signals may comprise one, two, three, four or even more individual signal components or distinct signals to make up said signalling. Some or all of these individual signals may be transmitted/received simultaneously, in sequence, and/or such that they temporally overlap one another.

With reference to any discussion of any mentioned computer and/or processor and memory (e.g. including ROM, CD-ROM etc.), these may comprise a computer processor, Application Specific Integrated Circuit (ASIC), field-programmable gate array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed examples may consist of any such individual feature or combination of features. In view of the foregoing description it will be evident to a person skilled in the art that various modifications may be made within the scope of the disclosure.

While there have been shown and described and pointed out fundamental novel features as applied to different examples thereof, it will be understood that various omissions and substitutions and changes in the form and details of the devices and methods described may be made by those skilled in the art without departing from the spirit of the invention. For example, it is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or example may be incorporated in any other disclosed or described or suggested form or example as a general matter of design choice. Furthermore, in the claims means-plus-function clauses are intended to cover the structures described herein as performing the recited function and not only structural equivalents, but also equivalent structures. Thus, although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. An apparatus comprising:
at least one processor; and
at least one memory including computer program code,
the at least one memory and the computer program code configured to, with the at least one processor, cause the apparatus to:
based on:
a received daily target activity level for a user; and
a received user daily activity profile for the user, the user daily activity profile comprising activity features each comprising specific intensity and specific duration at intervals throughout at least a portion of a day to cumulatively provide a particular activity level for the day, the user daily activity profile based on one or more of:
at least one previous user daily activity profile of the user; and
at least one previous user daily activity profile of one or more comparable users;
generate an adjusted user daily activity profile comprising adjusted activity features each comprising one or more of adjusted specific intensity and adjusted specific duration at intervals throughout at least a portion of a day to cumulatively provide the daily target activity level for the user; and
output the adjusted user daily activity profile for use by the user.

2. The apparatus of claim 1, wherein the apparatus is configured to use a machine-learning model to:
identify one or more of the activity features in the user daily activity profile as candidates for adjustment based on one or more of at least one previous user daily activity profile of the user; and at least one previous user daily activity profile of one or more comparable users; and
adjust the one or more identified candidate activity features, in accordance with the daily target activity level for the user, to generate the adjusted user daily activity profile.

3. The apparatus of any preceding claim, wherein the apparatus is configured to generate the adjusted user daily activity profile by:
adjusting one or more of the specific intensity and specific duration of the activity features of the user daily activity profile evenly over the at least a portion of a day, in accordance with the daily target activity level for the user, to provide an adjusted user daily activity profile.

4. The apparatus of any preceding claim, wherein the apparatus is configured to generate the adjusted user daily activity profile by adjusting activity features in the user daily activity profile to minimise the perceived change in daily activity by the user whilst still achieving the daily target activity level for the user.

5. The apparatus of any preceding claim, wherein the daily target activity level for the user indicates an increase or decrease in activity level compared to at least one of: a particular activity level of at least one previous user daily activity profile of the user, and a particular activity level of at least one previous user daily activity profile of one or more comparable users.

6. The apparatus of any preceding claim, wherein an adjusted activity feature of the adjusted user daily activity profile comprises one or more of:
the same specific intensity but an adjusted specific duration compared to a corresponding activity feature in the user daily activity profile;
the same specific duration but an adjusted specific intensity compared to a corresponding activity feature in the user daily activity profile; and
the same specific maximum intensity and same specific duration, but an adjusted gradient between minimum and maximum intensity levels of the adjusted activity feature compared to a corresponding activity feature in the user daily activity profile;
wherein an adjusted activity feature corresponds to an activity feature by one or more of:
each occurring at the same time of day;
each relating to the same activity type, and
the adjusted activity feature providing the same percentage contribution to the daily target activity level for the user as the activity feature provides to the particular activity level of the user daily activity profile.

7. The apparatus of any preceding claim, wherein the adjusted user daily activity profile comprises an adjusted activity feature at a particular time of day corresponding to a time of no activity in the user daily activity profile.

8. The apparatus of any preceding claim, wherein the apparatus is configured to:
generate a series of adjusted user daily activity profiles which progressively provide an intermediate daily target activity level for the user over a predetermined plurality of days, the intermediate daily target activity level for the user varying progressively over the predetermined plurality of days until it equals the daily target activity level for the user.

9. The apparatus of any preceding claim, wherein, dependent on the user daily activity profile for the user for a particular day, the apparatus is configured to adjust the daily target activity level for the user for a subsequent day to account for user activity recorded on the particular day by:
if the particular activity level for the particular day is higher than daily target activity level, the apparatus is configured to increase the daily target activity level for the subsequent day; and
if the particular activity level for the particular day is lower than daily target activity level, the apparatus is configured to decrease the daily target activity level for the subsequent day.

10. The apparatus of any preceding claim, wherein the user daily activity profile is based on at least one previous user daily activity profile of one or more comparable users and not based on at least one previous user daily activity profile of the user; and
wherein the apparatus is configured to use an objective function to identify and reduce an error between the at least one previous user daily activity profile of one or more comparable users and the generated adjusted user daily activity profile.

11. The apparatus of claim 10, wherein the objective function comprises one or more of:
a reproduction loss term configured to reduce an error between the generated adjusted user daily activity profile and a user daily activity profile received following output of the generated adjusted user daily activity profile;
a generative loss term configured to reduce an error in distributions between the at least one previous user daily activity profile of one or more comparable users and the generated adjusted user daily activity profile; and
a novel loss term configured to reduce an error between the particular activity level for the daily target activity level for the user obtained from the adjusted user daily activity profile, and the received daily target activity level.

12. The apparatus of any preceding claim, wherein the apparatus is configured to output the adjusted user daily activity profile for use by the user by one or more of:
providing for display of a graph indicating a current user daily activity profile and the target user daily activity profile;
providing a visual message prompting the user to one or more of perform or subsequently keep performing an upcoming activity in the adjusted user daily activity profile,
providing an audio message prompting the user to one or more of perform or subsequently keep performing an upcoming activity in the adjusted user daily activity profile, and
providing a haptic feedback (vibration etc.) prompting the user to one or more of perform or subsequently keep performing an upcoming activity in the adjusted user daily activity profile.

13. The apparatus of any preceding claim, wherein the user daily activity profile and the adjusted user daily activity profile provide an indication of the variation of one or more of the following parameters with time:
calories used,
heart rate,
steps taken,
distance travelled,
body temperature,
body hydration and perspiration,
breathing rate,
blood oxygenation,
blood-sugar level,
completion status of a task, and
time asleep.

14. A method comprising:
based on
a received daily target activity level for a user; and
a received user daily activity profile for the user, the user daily activity profile comprising activity features each comprising specific intensity and specific duration at intervals throughout at least a portion of a day to cumulatively provide a particular activity level for the day, the user daily activity profile based on one or more of:
at least one previous user daily activity profile of the user; and
at least one previous user daily activity profile of one or more comparable users;
generating an adjusted user daily activity profile comprising adjusted activity features each comprising one or more of adjusted specific intensity and adjusted specific duration at intervals throughout at least a portion of a day to cumulatively provide the daily target activity level for the user; and
outputting the adjusted user daily activity profile for use by the user.

15. A computer readable medium comprising computer program code stored thereon, the computer readable medium and computer program code being configured to, when run on at least one processor, perform the method of claim 14.
